(19) Europäisches Patentamt
European Patent Office
Office européen des brevets

(11) **EP 1 226 793 B1**

(12) **EUROPÄISCHE PATENTSCHRIFT**

(45) Veröffentlichungstag und Bekanntmachung des Hinweises auf die Patenterteilung:
**30.04.2008 Patentblatt 2008/18**

(51) Int Cl.:
***G05B 19/401*** *(2006.01)*    ***A61C 13/00*** *(2006.01)*

(21) Anmeldenummer: **02002246.3**

(22) Anmeldetag: **30.01.2002**

(54) **Verfahren zur Bestimmung aktueller Positionsdaten eines Bearbeitungswerkzeuges und Vorrichtung hierzu**

Method for determining the actual positional data of a tool and device therefor

Méthode pour la détermination de données de positionnement actuelles d'un outil, ainsi que dispositif associé

(84) Benannte Vertragsstaaten:
**AT BE CH CY DE DK ES FI FR GB GR IE IT LI LU MC NL PT SE TR**

(30) Priorität: **30.01.2001 DE 10104287**

(43) Veröffentlichungstag der Anmeldung:
**31.07.2002 Patentblatt 2002/31**

(73) Patentinhaber: **Sirona Dental Systems GmbH 64625 Bensheim (DE)**

(72) Erfinder: **Basler, Franz, Dipl.-Ing. 69514 Laudenbach (DE)**

(74) Vertreter: **Sommer, Peter Sommer Patentanwalt und European Patent and Trademark Attorney Augustaanlage 32 68165 Mannheim (DE)**

(56) Entgegenhaltungen:
**EP-A- 0 072 687**      **DE-A1- 4 030 175**

**Beschreibung**

Technisches Gebiet

Die Erfindung betrifft ein Verfahren zur Bestimmung aktueller Positionierdaten eines Bearbeitungswerkzeuges und eine Vorrichtung hierzu und ist insbesondere auf dem Gebiet der Herstellung von verwendungsfertigen Zahnrestaurations-Passkörpern mittels Schleifinstrumenten in dentalen CAD/CAM-Schleifmaschinen anzuwenden

Stand der Technik

Zur formschleifenden Bearbeitung von Keramikrohlingen sind Schleifstifte mit definiert geformter Oberfläche besonders geeignet. In einem automatisierten Herstellungsverfahren besteht dabei die Notwendigkeit, die Lage und Dimension des Schleifstiftes vor jedem Arbeitsgang zumindest aber nach Wechsel des Schleifstiftes zu vermessen. Diese Vermessung kann durch Antasten an ein Werkstück bekannter Größe erreicht werden. Der Antastvorgang besteht im Wesentlichen aus einem mit geringer Drehzahl rotierendem Schleifer und einer Bewegung des Schleifers gegen das Werkstück oder einer Bewegung des Werkstücks gegen den Schleifer. Der Antastvorgang wird durch den reibenden Kontakt zwischen Werkstück und Schleifer beendet, wenn der reibende Kontakt die Drehzahl des Schleifers auf Null verringert hat.

Aus der DE 40 30 175 C2 ist ein Verfahren zum kalibrieren eines motorisch angetriebenen und mit Hilfe einer Vorschubeinrichtung auf ein zu bearbeitendes Werkstück zu und von diesem wegbewegbaren Werkzeuges in Bezug auf das Werkstück bzw. einen das Werkstück aufnehmenden Halter sowie eine Vorrichtung zur Durchführung des Verfahrens bekannt.

Es hat sich gezeigt, dass das dort beschriebene Verfahren dann an seine Grenzen stößt, wenn die Form der Reibflächen äußerst undefiniert oder klein wird, was insbesondere bei zur Spitze sich verjüngenden Schleifern zu hohen Positionierungenauigkeiten beim axialen Antasten mit der Spitze führen kann.

Die Aufgabe der Erfindung besteht darin, die genauen linearen Positionierdaten eines Werkzeuges mit beliebiger Spitzengeometrie zu bestimmen.

Darstellung der Erfindung

Die Erfindung wird mit den Mitteln des Anspruch 1 gelöst. Das Bearbeitungswerkzeug wird langsam rotierend gegen ein senkrecht zur Vorschubsrichtung langsam rotierendes Werkstück mit bekannter Geometrie bewegt. Trifft das Werkzeug auf das Werkstück auf, wird die Rotation des Werkzeuges durch die Reibbelastung zwischen Werkstück und Werkzeug gestoppt. Zusätzlich zur Reibbelastung wirkt aufgrund der Werkstückrotation auf die Spitze des Bearbeitungswerkzeuges eine tangentiale Kraft, die zu erhöhten Reibkräften in der Lagerung der Antriebswelle des Werkzeuges führt, so dass die Position in Vorschubrichtung erheblich präziser bestimmt werden kann.

Es hat sich gezeigt, dass die Eindringtiefe und insbesondere die Varianz der Eindringtiefe des Werkzeugs in das Werkstück bis zum Stillstand des durch die Reibungskräfte abgebremsten Antriebs bei bestimmten Spitzengeometrien deutlich verringert werden kann, was sich unmittelbar in der Genauigkeit der Positionsbestimmung niederschlägt.

Vorteilhafte Ausgestaltungen der Erfindung sind in den Unteransprüchen angegeben.

Darüber hinaus weist eine Vorrichtung zur Durchführung des erfindungsgemäßen Verfahrens die Merkmale des Anspruchs 13 auf.

Bei der Vorrichtung ist eine Vorschubeinrichtung vorhanden, mit der das Werkzeug auf ein Werkstück zu und von diesem weg bewegt werden kann. Weiterhin ist eine erste Spindel zur Aufnahme des Werkstücks, mindestens eine weitere Spindel zur Aufnahme jeweils mindestens eines in Rotation versetzbaren Bearbeitungswerkzeuges mit einer stirnseitigen Bearbeitungsfläche, wobei die Spindeln so angeordnet und so gelagert sind, dass Bearbeitungswerkzeug und Werkstück im Sinne einer Materialabtragung am Werkstück aufeinander zu und von einander weg bewegt werden können, enthalten. Ferner sind Antriebsmotoren zur Verstellung der Spindeln und für den Antrieb des Bearbeitungswerkzeugs enthalten und mindestens eine am Werkstück, an der Werkstückhalterung oder an der Spannvorrichtung angeordnete Referenzfläche, gegen die die stirnseitige Bearbeitungsfläche des Bearbeitungswerkzeugs fährt, wobei bei Berührung der Referenzfläche ein den Vorschubweg des Werkzeuges festlegendes Signal erzeugt wird, welches zur Ermittlung der Startposition des Bearbeitungswerkzeuges verwendet wird. Darüber hinaus sind Antriebsmittel zum Bewegen der Referenzfläche an der stirnseitigen Bearbeitungsfläche des Bearbeitungswerkzeugs entlang während der durch den Vorschub bewirkten Berührung vorgesehen, und eine Berührungsfläche durch die stirnseitige Bearbeitungsfläche des Werkzeugs mit der Referenzfläche gebildet ist, wobei die Berührungsfläche asymmetrisch ist und Richtungskomponenten sowohl in Vorschubrichtung als auch in dazu radialer Richtung, allerdings nur über einen Teilumfang, aufweist.

Vorteilhafterweise weist das Werkzeug eine sich verjüngende Spitze auf und ist dabei insbesondere als Kugelschleifer oder Kegelschleifer mit Spitze ausgebildet.

Kurzbeschreibung der Zeichnung

In der Zeichnung ist ein Ausführungsbeispiel der Erfindung dargestellt. Es zeigt die

Fig. 1    eine Prinzipskizze eines Bearbeitungswerkzeuges mit einer Antriebswelle während des Berührvorganges an ein Werkzeug, die

Fig. 2    eine Detailansicht einer in eine Referenzfläche eingetauchten Werkzeugspitze im Querschnitt, die

Fig. 3    eine Vergrößerung der Fig. 2 zur Verdeutlichung der asymmetrischen Berührungsfläche des Werkzeugs mit der Referenzfläche, die

Fig. 4    eine Draufsicht auf die Referenzfläche mit einer eingetauchten Werkzeugspitze, die

Fig. 5    eine Vergrößerung der Werkzeugspitze aus Fig. 4 mit einem angenommenen Radialkraftverlauf am Werkzeug, die

Fig. 6    einen hypothetischen Kraftverlauf und die

Fig. 7    einen hypothetischen Momentenverlauf.

Ausführungsbeispiel der Erfindung

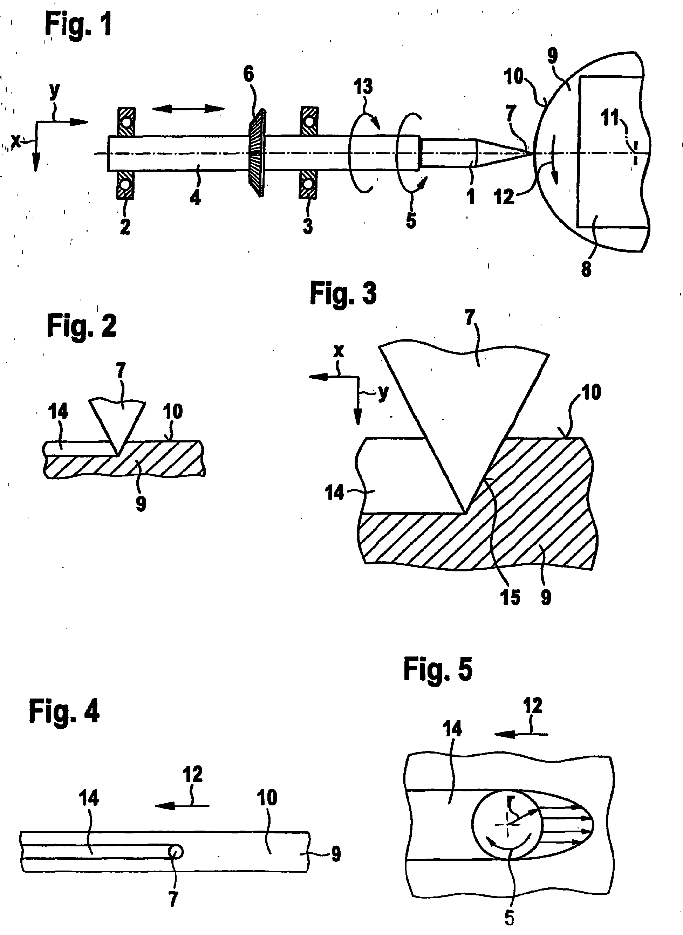

Die in Fig.1 dargestellte Prinzipskizze zeigt ein Bearbeitungswerkzeug 1 in Form eines Kegelschleifers, welches über eine in einem nicht dargestellten Gehäuse mittels eines ersten Lagers 2 und eines zweiten Lagers 3 gelagerte Antriebswelle 4 in Drehung gemäß Pfeil 5 versetzt wird. Dazu ist die Antriebswelle 4 mit einem zwischen dem ersten Lager 2 und dem zweiten Lager 3 angeordneten Zahnrad 6 verbunden, auf welches ein nicht dargestellter Antriebsmotor einwirkt.

Zu einer Spitze 7 des Bearbeitungswerkzeugs ist ein Werkstück 8, welches auf einem Halter 9 mit einer Referenzfläche 10 befestigt ist, in einem Abstand angeordnet. Die Referenzfläche 10 kann zusammen mit dem Halter 9 und dem Werkstück 8 um eine Drehachse 11 in Richtung des Pfeils 12 an der Spitze 7 vorbei bewegt werden.

Die gesamte Antriebswelle 4 und damit auch das Werkzeug 1 kann über nicht dargestellte Mittel auf das zu bearbeitende Werkstück 8 zu und von diesem weg bewegt werden.

Um die aktuellen Positionierdaten des Bearbeitungswerkzeuges 1 in Bezug auf das Werkstück 8 bzw. den das Werkstück 8 aufnehmenden Halter 9 zu bestimmen, weist im Ausführungsbeispiel der Halter 9 die Referenzfläche auf, an welche das Werkzeug mit seiner Spitze zur Berührung gebracht wird.

Dabei wird die Drehzahl des Antriebsmotors für das Werkzeug 1 auf eine Anfahrdrehzahl eingestellt die so niedrig ist, dass bei einer Berührung der Referenzfläche 10 durch die Werkzeugspitze 7 aufgrund der dadurch hervorgerufenen Reibungskräfte die Drehzahl des Antriebsmotors zu Null wird. Der Antriebsmotor wird also reibungsbedingt bis zum Stillstand abgebremst.

Um die Ausbildung von Reibungskräften an einer punktförmig zulaufenden Spitze 7 zu unterstützen, wird das Werkstück 8 bzw. der das Werkstück 8 aufnehmende Halter 9 bereits vor und auch noch während der durch den Vorschub bewirkten Berührung durch die Werkzeugspitze 7 an dieser vorbei bewegt. Hierzu sind nicht dargestellte Antriebsmittel vorgesehen.

Bereits durch die allein vom Vorschub des Werkzeugs 1 hervorgerufene Berührung der Spitze 7 mit der Referenzfläche 10 entsteht aufgrund der Reibung und in der Regel aufgrund eines Materialabtrags an der Referenzfläche durch die Spitze selbst ein dem Antriebsmoment 5 entgegengesetztes Bremsmoment in Richtung des Pfeils 13. Es ist unmittelbar einleuchtend, dass dieses Bremsmoment bei einer kegelförmig zulaufenden Spitze am Anfang aufgrund der nahezu punktförmigen Berührungsfläche sehr klein bleiben muß, sodass erst bei weiterem Vorschub der Werkzeugspitze und bei einem entsprechend tiefen Eindringen in die Referenzfläche ein ausreichend großes Bremsmoment aufgebaut wird.

Zusätzlich zu der durch den Vorschub bewirkten Berührung und dem daraus entstehenden Bremsmoment wird nun durch die aus der Bewegung der Referenzfläche an der Spitze entlang entstehende Querkraft in den Lagern der Antriebswelle ein weiteres Bremsmoment aufgebaut, wodurch die Antriebswelle deutlich früher zum Stillstand kommt als ohne eine Bewegung der Referenzfläche.

In Fig. 2 ist eine Detailansicht einer in eine Referenzfläche 10 eingetauchten Werkzeugspitze 7 im Querschnitt gezeigt. Dabei ist zu erkennen, dass sich durch das Eintauchen der Werkzeugspitze und die Bewegung der Referenzfläche 10 an der Werkzeugspitze 7 vorbei eine Nut 14 ausgebildet hat.

In Fig. 3 ist zu erkennen, dass die Werkzeugspitze 7 eine Berührungsfläche 15 mit dem Halter 9 aufweist, welche wegen der gebildeten Nut 14 asymmetrisch ist und sowohl axiale als auch radiale Richtungskomponenten (y-Richtung und x-Richtung) aufweist.

In Fig. 4 ist eine Draufsicht auf die Referenzfläche 10 mit einer eingetauchten Werkzeugspitze 7 gezeigt, aus der die Bewegung, dargestellt durch den Pfeil 12, der Referenzfläche 10 an der Werkzeugspitze 7 vorbei verdeutlicht ist. Die Referenzfläche 10 wurde dabei in eine Ebene abgewickelt. Aufgrund der in die Referenzfläche 10 eingetauchten Werkzeugspitze 7 ist die Nut 14 entstanden, bevor die Bremsmomente den Antriebsmotor abgebremst haben.

Bei abgebremstem Antriebsmotor wird dafür Sorge getragen, dass sich die Referenzfläche 10 nicht weiterdrehen kann. Dies wird dadurch bewirkt, dass die Drehzahl des Bearbeitungswerkzeugs bzw. des Antriebsmotors als Führungsgröße für die Bewegung der Referenzfläche verwendet wird. Sinkt die Drehzahl des Werkzeugs, so verringert sich die Bewegung der Referenzfläche. Wird das Werkzeug bis auf Null abgebremst, so wird auch die Bewegung der Referenzfläche zu Null. Es ist darüber hinaus möglich, auch den Vorschub des Werkzeugs an die Drehzahl des Werkzeugs zu koppeln, in dem bei sich verringernder Drehzahl des Werkzeugs auch der Vorschub des Werkzeugs auf die Referenzfläche zu verringert wird. Wird die Drehung des Bearbeitungswerkzeugs aufgrund der auftretenden Reibung gleich Null, so erfolgt auch kein Vorschub des Bearbeitungswerkzeugs mehr.

Darüber hinaus kann in einer Vorschubeinrichtung der Antriebswelle oder an der Antriebswelle selbst ein vorgespanntes Federelement vorgesehen sein, welches durch Einfedern unter Last die auf das stillstehende Werkzeug einwirkenden Kräfte aufnimmt. Das Einfedern erfolgt dabei vorzugsweise gegen die Richtung des Vorschubs, wobei die Vorspannung des Federelements groß genug gewählt werden muß um die während der Bearbeitung planmäßig auftretenden vorgesehenen Belastungen des Werkzeugs zu übertreffen. Ein Einfedern findet daher nur zum Schutz des Werkzeugs gegen Überlast statt. Dadurch kann einerseits eine Beschädigung der Werkzeugspitze durch Überlast vermieden werden und andererseits eine genaue Bearbeitung des Werkstücks mit der Werkzeugspitze erfolgen.

In Fig. 5 ist eine Vergrößerung der Werkzeugspitze 7 aus Fig. 4 mit einem angenommenen Radialkraftverlauf an der Werkzeugspitze 7 gezeigt. Zu jeder Radialkraft entsteht eine der Drehrichtung gemäß Pfeil 5 entgegengesetzte Reibkraft, die tangential an die Mantelfläche der Spitze 7 anliegt und zum einen ein Bremsmoment mit dem Hebelarm r erzeugt, zum anderen durch die asymmetrische Verteilung Lagerkräfte an der Antriebswelle hervorruft.

Während der Bewegung des Werkzeugs auf das Werkstück zu legt die Referenzfläche des Werkstücks einen Weg zurück, der höchstens das Zehnfache der gewünschten Genauigkeit der Bearbeitung beträgt, vorzugsweise weniger als 0,2 mm. Damit soll dafür Sorge getragen werden, dass Exzentrizitäten der Referenzfläche, die auf andere Weise bestimmt wurden und bekannt sind, die Genauigkeit der Positionserfassung der Werkzeugspitze nicht beeinträchtigen. Bei einer zylindrischen Referenzfläche wirken sich kleine Abweichungen bei der Ausrichtung des Werkzeugs auf die Mittelachse der Referenzfläche nur geringfügig aus. Gleichwohl sollte die Lage der Mittelachse einer zylindrischen Referenzfläche vorher genau bestimmt worden sein und eine Berührung mit einer mit der Mittelachse fluchtenden Vorschubrichtung erfolgen.

Es hat sich gezeigt, dass es bei der Positionsbestimmung eines diamantierten Schleifers an einer Referenzfläche aus Aluminium ausreicht, wenn das Bearbeitungswerkzeug vor der Berührung mit einer Drehzahl von weniger als einem Hunderstel der zur Bearbeitung verwendeten Drehzahl angetrieben ist, insbesondere mit 1 bis 10 Umdrehungen/Sekunde.

Das Werkzeug 1 und die Referenzfläche 10 werden vor dem eigentlichen Berühren zur Positionsbestimmung der Werkzeugspitze 7 zueinander ausgerichtet, wobei es sich hier um eine grobe Bestimmung der Position bezüglich des Abstandes zur Referenzfläche 10 handelt. Erst nach dieser groben Lagenbestimmung, die mit gegenüber der sich anschließenden Positionsbestimmung deutlich größeren Vorschubgeschwindigkeiten erfolgen kann, findet ein langsames Zustellen des Werkzeugs auf die Referenzfläche statt, sodass insgesamt eine kürzere Zeit benötigt wird.

Das Bearbeitungswerkzeug 1 wird bezüglich der Vorschubachse fluchtend zu einem Drehmittelpunkt 11 einer zylindrischen Referenzfläche 10 ausgerichtet. Wenn die genaue Lage des Drehmittelpunktes der Referenzfläche zu diesem Zeitpunkt noch nicht bekannt ist kann sie dadurch bestimmt werden, dass das Werkzeug in Vorschubrichtung bis zur Berührung der Referenzfläche 10 zugestellt wird. Aus dem bekannten Durchmesser der Referenzfläche lässt sich dann der absolute Abstand des Werkzeugs zum Drehmittelpunkt ermitteln. Um Exzentrizitäten zu berücksichtigen kann es erforderlich sein, eine weitere Berührung an einer vorzugsweise um 180° gedrehten Referenzfläche vorzunehmen.

Vor der eigentlichen Positionsbestimmung werden die den Drehmittelpunkt 11 der Referenzfläche 10 enthaltende und in oder parallel zur Vorschubsrichtung sich erstreckende Ebene und die Exzentrizitäten der Referenzfläche 10 festgestellt. Dazu kann das Bearbeitungswerkzeug 1 seitlich an die Referenzfläche gebracht werden. Es müssen mindestens zwei Stellen seitlich angefahren werden, um unter Verwendung des Durchmessers der Referenzfläche die Kreisgleichung aufstellen zu können. Weitere Stellen ermöglichen Aussagen über die Exzentrizitäten in verschiedenen Richtungen.

Vor der eigentlichen Positionsbestimmung des Werkzeuges 1 bezüglich der Referenzfläche 10 können ein oder mehrere Antastvorgänge mit stillstehender Referenzfläche 10 erfolgen.

Nach der Positionsbestimmung kann die festgestellte Position um einen vorgegebener Korrekturwert verändert werden, beispielsweise um trägheitsbedingte Systemeigenschaften zu berücksichtigen. Dadurch kann sich die Genauigkeit nochmals verbessern.

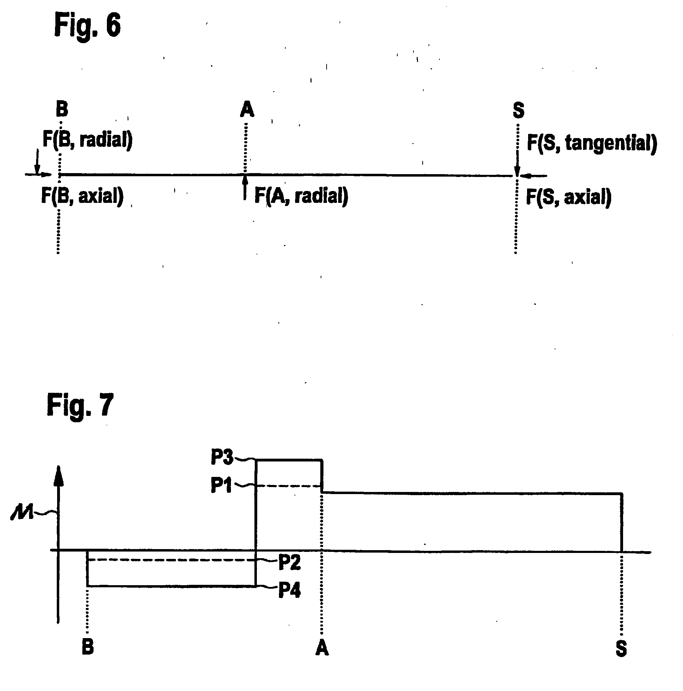

In Fig. 6 ist ein hypothetischer Kraftverlauf und in Fig. 7 ein hypothetischen Momentenverlauf am Beispiel eines Schleifstiftes mit Kegelspitze dargestellt. Vor jedem Schleifvorgang wird der Schleifer axial zum Drehzentrum eines zylindrischen Werkstücks positioniert. Es wird darauf geachtet, dass der Abstand zwischen Schleiferspitze und Werkstück sehr kurz ist, damit nur kurze Antastwege zu fahren sind und der Einfluss der Exzentrizität des Werkstücks, die sich bei der Drehung des Werkstücks bemerkbar macht, vernachlässigbar klein wird. Dies kann durch einen vorherigen groben Antastvorgang mit stillstehendem Werkstück geschehen.

Danach wird der Schleifer in langsame Rotation (1-10 Hz) versetzt. Dabei wird dem Antriebsmotor, der mit einem Zahnrad 6 in die Antriebswelle 4 eingreift, gerade soviel Leistung zugeführt, dass die Reibmomente der realen Lagerung und des Getriebefetts überwunden werden. Die hervorgerufene Drehzahl des Schleifers wird dabei so niedrig angesetzt, dass eine Vorschädigung des Schleifers ausgeschlossen werden kann.

Jetzt wird das Werkstück in langsame Drehung versetzt und der Schleifer in axialer Richtung auf das Werkstück zu bewegt. Die Drehzahl des Werkstücks ist dabei sehr klein gewählt, so dass die Spitze des Schleifers nur einen geringen Teilumfang des Werkstückes auf dem Vorschubweg überstreicht. Damit wird der Einfluss der Exzentrizität des Werkstücks vernachlässigbar.

Bei Kontakt des Schleifers mit dem Werkstück entsteht durch die diamantierte Schleiferspitze ein Reibmoment zwischen Diamant und Werkstückoberfläche, das der Rotation des Schleifers entgegenwirkt. Der Stillstand des Schleifers kann dann über Sensoren auf der Motorwelle detektiert werden, die aktuelle Vorschubposition kann gespeichert werden.

Zusätzlich zur von dem Schleifer mittels Reibung hervorgerufenen, in axialer Richtung wirkenden Kraft F(S, axial), die ein geringes Bremsmoment auf die Welle einleitet, wirkt auch eine durch die Drehung der Referenzfläche hervorgerufene tangentiale Kraft F(S,tangential). Daraus resultieren zusätzlich Kräfte F in den Lagern A und B bzw. 2 und 3 aus Fig. 1. Es ergibt sich folgende Situation:

$$F_{(B,axial)} - F_{(S,axial)} = 0$$

$$F_{(B,radial)} - F_{(A,radial)} + F_{(S,tangential)} = 0$$

Für das entstehende Moment gilt:

$$F_{(B,radial)} * Abstand(B,A) = F_{(S,tangential)} * Abstand(A,S)$$

Damit ergeben sich die zusätzlichen radialen Kraftkomponenten in den Lagern A,B:

$$F_{(B,radial)} = F_{(S,tangential)} * (Abstand(A,S) / Abstand(B,A))$$

$$F_{(A,radial)} = F_{(S,tangential)} * (1 + Abstand(A,S) / Abstand(B,A))$$

Diese radialen Kraftkomponenten erzeugen über den Reibwert ein zusätzliches Bremsmoment auf das in Fig. 1 abgebildete Zahnrad 5 und den Antriebsmotor.

Im skizzierten Momentenverlauf gemäß Fig. 7 entspricht die Differenz des Moments der Punkte P1-P2 dem erzeugten Bremsmoment ohne Werkstückrotation und die Differenz P3-P4 dem erzeugten Bremsmoment mit Werkstückrotation, also mit tangentialer Kraft F(S,tangential).

**Patentansprüche**

1. Verfahren zur Bestimmung aktueller Positionierdaten eines Bearbeitungswerkzeuges (1) in Bezug auf eine an einem Werkstück (8) bzw. an einem mit dem Werkstück verbundenen Körper (9) vorhandene Referenzfläche (10), wobei das Werkzeug und/oder das die Referenzfläche umfassende zu bearbeitende Werkstück zum Herstellen einer Berührung des Werkzeugs an der Referenzfläche aufeinander zu bewegbar sind, wobei die Drehzahl eines Antriebsmotors für das Werkzeug auf eine Anfahrdrehzahl eingestellt wird, die so niedrig ist, dass bei einer Berührung der Referenzfläche durch das Werkzeug dessen Drehzahl messbar verringert wird und Positionierdaten bestimmbar sind, **dadurch gekennzeichnet, daß** die Referenzfläche (10) vor und während der Berührung mit dem Werkzeug (1) an dem Werkzeug (1) vorbei bewegt wird.

2. Verfahren nach Anspruch 1, **dadurch gekennzeichnet, dass** die Referenzfläche (10) während der Bewegung des Werkzeugs (1) auf das Werkstück (8) zu einen Weg zurücklegt, der höchstens das Zehnfache der gewünschten Genauigkeit der Bearbeitung, vorzugsweise weniger als 0,2 mm beträgt.

3. Verfahren nach einem der Ansprüche 1 oder 2, **dadurch gekennzeichnet, dass** das Bearbeitungswerkzeug (1) vor der Berührung mit einer Drehzahl von weniger als einem Hunderstel der zur Bearbeitung verwendeten Drehzahl, insbesondere mit einer Drehzahl von 1 bis 10 Umdrehungen/Sekunde angetrieben ist.

4. Verfahren nach einem der Ansprüche 1 bis 3, **dadurch gekennzeichnet, dass** das Werkzeug (1) und die Referenzfläche (10) vor der eigentlichen Positionsbestimmung zueinander ausgerichtet werden.

5. Verfahren nach einem der Ansprüche 1 bis 4, **dadurch gekennzeichnet, dass** das Bearbeitungswerkzeug (1) bezüglich der Vorschubachse fluchtend zu einem Drehzentrum (11) einer zylindrischen Referenzfläche (10) ausgerichtet wird.

6. Verfahren nach Anspruch 5, **dadurch gekennzeichnet, dass** vor der eigentlichen Positionsbestimmung die den Drehmittelpunkt (11) der Referenzfläche (10) enthaltende und in oder parallel zur Vorschubsrichtung sich erstreckende Ebene und die Exzentrizitäten der Referenzfläche (10) festgestellt werden.

7. Verfahren nach einem der Ansprüche 4 bis 6, **dadurch gekennzeichnet, dass** vor der eigentlichen Positionsbestimmung des Werkzeuges (1) bezüglich der Referenzfläche (10) ein oder mehrere Antastvorgänge mit stillstehender Referenzfläche (10) erfolgen.

8. Verfahren nach einem der Ansprüche 1 bis 7, **dadurch gekennzeichnet, dass** die Bewegung der Referenzfläche (10) so an die Drehzahl des Antriebsmotors für das Werkzeug (1) gekoppelt ist, dass eine Verringerung der Drehzahl des Antriebsmotors eine Verringerung der Bewegung der Referenzfläche (10) bewirkt.

9. Verfahren nach einem der Ansprüche 1 bis 8, **dadurch gekennzeichnet, dass** der Vorschub des Werkzeugs (1) so an die Drehzahl des Antriebsmotors für das Werkzeug (1) gekoppelt ist, dass eine Verringerung der Drehzahl des Antriebsmotors eine Verringerung des Vorschubs des Werkzeugs bewirkt.

10. Verfahren nach Anspruch 8 und/oder 9, **dadurch gekennzeichnet, dass** die Bewegung der Referenzfläche (10) bzw. des Vorschubs des Werkzeugs (1) angehalten wird, wenn die Drehzahl des Antriebsmotors des Werkzeugs Null wird.

11. Verfahren nach einem der Ansprüche 1 bis 10, **dadurch gekennzeichnet, dass** während des Herantastens der Werkzeugspitze (7) an die Referenzfläche (10) dem Antriebsmotor so viel Leistung zugeführt wird, dass die Reibmomente der realen Lagerung und aller Schmierungen gerade überwunden werden.

12. Verfahren nach einem der Ansprüche 1 bis 11, **dadurch gekennzeichnet, dass** eine Position festgestellt und zur weiteren Verbesserung der Genauigkeit um einen vorgegebenen trägheitsbedingte Systemeingenschaften berücksichtigenden Korrekturwert verändert wird.

13. Vorrichtung zur Durchführung des Verfahrens nach einem der Ansprüche 1 bis 12, wobei eine Vorschubeinrichtung vorhanden ist, mit der das Werkzeug (1) auf ein Werkstück (8) zu und von dieser weg bewegt werden kann, enthaltend eine erste Spindel zur Aufnahme des Werkstücks (8), mindestens eine weitere Spindel zur Aufnahme jeweils mindestens eines in Rotation versetzbaren Bearbeitungswerkzeuges (1) mit einer stirnseitigen Bearbeitungsfläche (7), wobei die Spindeln so angeordnet und so gelagert sind, dass Bearbeitungswerkzeug (1) und Werkstück (8) im Sinne einer Materialabtragung am Werkstück (8) aufeinander zu und von einander weg bewegt werden können, enthaltend ferner Antriebsmotoren zur Verstellung der Spindeln und für den Antrieb des Bearbeitungswerkzeugs, enthaltend ferner mindestens eine am Werkstück (8), an der Werkstückhalterung (9) oder an einem mit dem Werkstück verbundenen Körper angeordnete Referenzfläche (10), gegen die die stirnseitige Bearbeitungsfläche (7) des Bearbeitungswerkzeug (1) fährt, wobei bei Berührung der Referenzfläche ein den Vorschubweg des Werkzeuges festlegendes Signal erzeugt wird, welches zur Ermittlung der Startposition des Bearbeitungswerkzeuges (1) verwendet wird, **dadurch gekennzeichnet, dass** Antriebsmittel zum Bewegen der Referenzfläche (10) an der stirnseitigen Bearbeitungsfläche des Bearbeitungswerkzeugs (1) entlang während der durch den Vorschub bewirkten Berührung vorgesehen sind und dass eine Berührungsfläche (15) durch die stirnseitige Bearbeitungsfläche des Werkzeugs (1) mit der Referenzfläche (10) gebildet ist, wobei die Berührungsfläche (15) asymmetrisch ist und Richtungskomponenten sowohl in Vorschubrichtung als auch in dazu radialer Richtung, allerdings nur über einen Teilumfang, aufweist.

**14.** Vorrichtung nach Anspruch 13, **dadurch gekennzeichnet, dass** das Werkzeug eine sich verjüngende Spitze aufweist und insbesondere als Kugelschleifer oder Kegelschleifer mit Spitze ausgebildet ist.

**15.** Vorrichtung nach Anspruch 13 oder 14, **dadurch gekennzeichnet, dass** die Antriebsmittel zum Bewegen der Referenzfläche an die Drehzahl des Bearbeitungswerkzeugs so gekoppelt sind, dass eine Verringerung der Drehzahl des Bearbeitungswerkzeugs die durch die Antriebsmittel hervorgerufene Bewegung verringert.

**Claims**

**1.** A method for acquiring current position data of a machining tool (1) relative to a reference surface (10) on a workpiece (8) or on a body (9) connected to said workpiece, wherein the tool and/or the workpiece to be machined comprising said reference surface are capable of being moved toward each other to cause the tool to touch the reference surface, and the speed of rotation of a drive motor for the tool is adjusted to a speed of approach which is so low that when the reference surface is touched by the tool the speed of rotation thereof is measurably reduced and position data can be acquired, **characterized in that** said reference surface (10) is moved past said tool (1) prior to and during the contact with said tool (1).

**2.** The method according to claim 1, **characterized in that** during the movement of the tool (1) toward said workpiece (8) said reference surface (10) moves through a distance that is not more than ten times the desired machining accuracy and is preferably less than 0.2 mm.

**3.** The method according to claim 1 or claim 2, **characterized in that** said machining tool (1) is driven, prior to making contact, at a speed of rotation of less than one hundredth of the speed of rotation used for machining, and in particular has a speed of rotation of from 1 to 10 rps.

**4.** The method according to any one of claims 1 to 3, **characterized in that** said tool (1) and said reference surface (10) are mutually aligned prior to detection of the position of said tool.

**5.** The method according to any one of claims 1 to 4, **characterized in that** the feed axis of said machining tool (1) is oriented to be on a level with a fulcrum (11) of a cylindrical reference surface (10).

**6.** The method according to claim 5, **characterized in that** prior to detecting the position of said tool, the plane enclosing said fulcrum (11) of said reference surface (10) and extending in, or parallel to, the feed direction and the eccentricities of said reference surface (10) are determined.

**7.** The method according to any one of claims 4 to 6, **characterized in that**, prior to detection of the current position of said tool (1) with reference to said reference surface (10), one or more touching operations are carried out while said reference surface (10) is stationary.

**8.** The method according to any one of claims 1 to 7, **characterized in that** the motion of said reference surface (10) is governed by the speed of rotation of the drive motor for said tool (1) such that a reduction in the speed of rotation of the drive motor decelerates the motion of said reference surface (10).

9.   The method according to any one of claims 1 to 8, **characterized in that** the rate of feed of said tool (1) is dependent on the speed of rotation of the drive motor for said tool (1) such that a reduction in the speed of rotation of said drive motor decelerates the rate of feed of said tool.

10.  The method according to claim 8 and/or claim 9, **characterized in that** the motion of said reference surface (10) or the feed of said tool (1) is stopped if the speed of rotation of said drive motor for said tool becomes zero.

11.  The method according to any one of claims 1 to 10, **characterized in that** when the tool tip (7) touches said reference surface (10), said drive motor receives just enough power to permit it to overcome the moments of friction of the physical bearings and all lubrication means.

12.  The method according to any one of claims 1 to 11, **characterized in that** a position is detected and is modified by a specific inertia-dependent correction value allowing for the properties of the system, in order to achieve further accuracy improvement.

13.  A device for carrying out the method according to any one of claims 1 to 12, wherein a feed system is provided, by means of which said tool (1) can be moved toward, and away from, a workpiece (8), comprising a first spindle for the accommodation of said workpiece (8), one or more further spindles each for the accommodation of at least one rotatable machining tool (1) having a cutting surface (7) disposed at its end, and said spindles are configured and mounted such that said machining tool (1) and said workpiece (8) can be moved toward, and away from, each other as if intended to remove material from said workpiece (8), further comprising drive motors for the adjustment of said spindles and for driving said machining tool, further comprising at least one reference surface (10) disposed on said workpiece (8), on the workpiece holder (9), or on a body connected to said workpiece, toward which the end-cutting surface (7) of the machining tool (1) advances, and when contact with the reference surface occurs, a signal indicating a feed increment of said tool is produced, which is used for defining the start position of said machining tool (1), **characterized in that** driving means are provided for moving said reference surface (10) across said end-cutting surface of said machining tool (1) during the period of contact caused by said feed increment and that a contact area (15) is formed on said reference surface (10) by said end-cutting surface of said tool (1), which contact area (15) is asymmetrical and has direction components both in the direction of feed and, to a partial extent, in the direction radial thereto.

14.  The device according to claim 13, **characterized in that** said tool has a tapered tip and is, in particular, in the form of a spherical grinder or a pointed conical grinder.

15.  The device according to claim 13 or claim 14, **characterized in that** said driving means for moving said reference surface are dependent on the speed of rotation of said machining tool such that a reduction in the speed of rotation of said machining tool decelerates the motion produced by said driving means.

**Revendications**

1.   Procédé pour déterminer des données de positionnement actuelles d'un outil d'usinage (1) par rapport à une surface de référence (10) présente sur une pièce à usiner (8) ou sur un corps (9) relié à la pièce à usiner, l'outil et/ou la pièce à usiner comprenant la surface de référence étant mobiles l'un vers l'autre, pour établir un contact de l'outil sur la surface de référence, le régime d'un moteur d'entraînement pour l'outil étant réglé à un régime de démarrage qui est si faible, que lors d'un contact de l'outil sur la surface de référence, sa vitesse de rotation se réduit de façon mesurable et les données de positionnement peuvent être déterminées, **caractérisé en ce que**, pendant le contact avec l'outil (1), la surface de référence (10) se déplace sur l'avant de l'outil (1).

2.   Procédé selon la revendication 1, **caractérisé en ce que** pendant le déplacement de l'outil (1) vers la pièce à usiner (8), la surface de référence (10), parcourt une course correspondant au maximum à dix fois la précision d'usinage souhaitée, s'élevant de préférence à moins de 0,2 mm.

3.   Procédé selon l'une quelconque des revendications 1 ou 2, **caractérisé en ce que**, avant le contact, l'outil d'usinage (1) est entraîné à une vitesse de rotation inférieure à un centième de la vitesse de rotation utilisée pour l'usinage, notamment à une vitesse de rotation de 1 à 10 tour(s)/seconde.

4.   Procédé selon l'une quelconque des revendications 1 à 3, **caractérisé en ce** l'outil (1) et la surface de référence

**EP 1 226 793 B1**

(10) sont orientés l'une par rapport à l'autre avant la détermination réelle de la position.

5. Procédé selon l'une quelconque des revendications 1 à 3, **caractérisé en ce** l'outil d'usinage (1) est orienté par rapport à l'axe d'avance, en alignement sur un centre de rotation (11) d'une surface de référence (10) cylindrique.

6. Procédé selon la revendication 5, **caractérisé en ce que**, avant la détermination réelle de la position, le plan comportant le point de rotation central (11) de la surface de référence (10) et/ou s'étendant à la parallèle de la direction d'avance et les excentricités de la surface de référence (10) sont fixés.

7. Procédé selon l'une quelconque des revendications 4 à 6, **caractérisé en ce que**, avant la détermination réelle de la position de l'outil (1) par rapport à la surface de référence (10), on procède à une ou à plusieurs opérations de palpage, la surface de référence (10) étant à l'arrêt.

8. Procédé selon l'une quelconque des revendications 1 à 7, **caractérisé en ce que** le déplacement de la surface de référence (10) est couplé sur le régime du moteur d'entraînement pour l'outil (1), de sorte qu'une diminution de régime du moteur d'entraînement provoque une diminution du déplacement de la surface de référence (10).

9. Procédé selon l'une quelconque des revendications 1 à 8, **caractérisé en ce que** l'avance de l'outil (1) est couplée sur le régime du moteur d'entraînement pour l'outil (1), de sorte qu'une diminution de régime du moteur d'entraînement provoque une diminution de l'avance de l'outil.

10. Procédé selon la revendication 8 et/ou 9, **caractérisé en ce que** le déplacement de la surface de référence (10), respectivement de l'avance de l'outil (1) s'arrête lorsque le régime du moteur d'entraînement de l'outil est à zéro.

11. Procédé selon l'une quelconque des revendications 1 à 10, **caractérisé en ce** pendant le palpage de contact de la pointe de l'outil (7) sur la surface de référence (10), il est alimenté vers le moteur une puissance telle, que les couples de frottement du logement réel et de tous les graissages sont juste surmontés.

12. Procédé selon l'une quelconque des revendications 1 à 11, **caractérisée en ce qu'**une position est déterminée, et pour améliorer encore la précision, elle est modifiée du montant d'une valeur de correction tenant compte des caractéristiques d'inertie du système.

13. Dispositif pour la réalisation du procédé selon l'une quelconque des revendications 1 à 12, un dispositif d'avance étant présent, à l'aide duquel l'outil (1) peut être déplacé vers une pièce à usiner (8) et à partir de cette dernière, comprenant une première broche pour recevoir la pièce à usiner (8), au moins une broche supplémentaire pour recevoir chaque fois un outil d'usinage (1) susceptible d'être amené en rotation, avec une surface d'usinage (7) frontale, les broches étant disposées et logées de sorte que l'outil d'usinage (1) et la pièce à usiner (8) peuvent être déplacés l'un vers l'autre ou l'un à partir de l'autre dans le sens d'un enlèvement de matière sur la pièce à usiner (8), comprenant en outre des moteurs d'entraînement pour le déplacement des broches et pour l'entraînement de l'outil d'usinage, comprenant en outre au moins une surface de référence (10) disposée sur la pièce à usiner (8), sur la fixation de la pièce à usiner (9) ou sur un corps relié à la pièce à usiner, contre laquelle la surface d'usinage (7) frontale de l'outil d'usinage (1) se déplace, lors d'un contact sur la surface de référence, un signal définissant la course d'avance de l'outil, qui est utilisé pour rechercher la position de départ de l'outil d'usinage (1) étant délivré, **caractérisé en ce qu'**il est prévu des moyens d'entraînement pour le déplacement de la surface de référence (10) le long de la surface d'usinage frontale de l'outil d'usinage (1) pendant le contact provoqué par l'avance et **en ce qu'**une surface de contact (15) est formée par la surface d'usinage frontale de l'outil (1) avec la surface de référence (10), la surface de contact (15) étant asymétrique et comportant des composantes directionnelles, aussi bien en direction d'avance, qu'en direction radiale par rapport à cette dernière, mais toutefois seulement sur une périphérie partielle.

14. Dispositif selon la revendication 13, **caractérisé en ce que** l'outil comporte une pointe se rétrécissant et notamment **en ce qu'**il est conçu sous la forme d'un outil d'affûtage sphérique ou conique avec pointe.

15. Dispositif selon la revendication 13 ou 14, **caractérisé en ce que** les moyens d'entraînement pour le déplacement de la surface de référence sont couplés à la vitesse de rotation de l'outil d'usinage, de sorte qu'une diminution de la vitesse de rotation de l'outil d'usinage diminue le déplacement provoqué par les moyens d'entraînement.

9

**Fig. 1**

**Fig. 2**

**Fig. 3**

**Fig. 4**

**Fig. 5**

## Fig. 6

B          A          S

F(B, radial)                      F(S, tangential)

F(B, axial)        F(A, radial)           F(S, axial)

## Fig. 7

P3

P1

M

P2

P4

B            A           S

## EP 1 226 793 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4030175 C2